# EUROPÄISCHE PATENTSCHRIFT

(11) **EP 1 373 898 B1**
(45) Veröffentlichungstag und Bekanntmachung des Hinweises auf die Patenterteilung: **28.06.2006**
(21) Anmeldenummer: 02730067.2
(22) Anmeldetag: 26.03.2002
(51) Int. Cl.: G01N 33/573

(54) **NACHWEIS VON ENTZÜNDUNGEN**
DETECTION OF INFLAMMATIONS
DETECTION D'INFLAMMATIONS

(30) Priorität: 27.03.2001 DE 10115083
(43) Veröffentlichungstag der Anmeldung: 02.01.2004
(73) Patentinhaber: ScheBo Biotech Aktiengesellschaft, 35394 Giessen (DE)
(72) Erfinder: SCHLATTERER, Bert, 14532 Kleinmachnow (DE); BAEKKER, Regine, 14469 Potsdam (DE); SCHEEFERS-BORCHEL, Ursula, 35394 Giessen (DE); SCHEEFERS, Hans, 35394 Giessen (DE)
(74) Vertreter: Dey, Michael
(86) Internationale Anmeldenummer: PCT/EP2002/003383
(87) Internationale Veröffentlichungsnummer: WO 2002/077643

(56) Entgegenhaltungen:
- EP-A- 0 913 405
- CA-A- 2 225 270
- DE-A- 19 744 132
- DE-A- 19 801 243
- US-A- 5 270 163
- DORTA-CONTRERAS A J ET AL: "Beta-trace protein in the cerebrospinal fluid and serum in meningoencephalitis" REVISTA DE NEUROLOGIA 1998 SPAIN, Bd. 26, Nr. 151, 1998, Seiten 386-388, XP001121347 ISSN: 0210-0010
- BAEKER R ET AL: "Lipocalin-type prostaglandin D synthase in milk: a new biomarker for bovine mastitis" PROSTAGLANDINS, BUTTERWORTH, STONEHAM, MA, US, Bd. 67, Nr. 1, Januar 2002 (2002-01), Seiten 75-88, XP004330580 ISSN: 0090-6980
- LOGDBERG^A L ET AL: "Immunocalins: a lipocalin subfamily that modulates immune and inflammatory responses" BIOCHIMICA ET BIOPHYSICA ACTA. PROTEIN STRUCTURE AND MOLECULAR ENZYMOLOGY, ELSEVIER, AMSTERDAM,, NL, Bd. 1482, Nr. 1-2, 18. Oktober 2000 (2000-10-18), Seiten 284-297, XP004279081 ISSN: 0167-4838
- XU S ET AL: "Lipocalins as biochemical markers of disease" BIOCHIMICA ET BIOPHYSICA ACTA. PROTEIN STRUCTURE AND MOLECULAR ENZYMOLOGY, ELSEVIER, AMSTERDAM,, NL, Bd. 1482, Nr. 1-2, 18. Oktober 2000 (2000-10-18), Seiten 298-307, XP004279082 ISSN: 0167-4838
- ATROSHI F ET AL: "POSSIBLE ROLES OF VITAMIN E AND GLUTATHIONE METABOLISM IN BOVINE MASTITIS" INTERNATIONAL JOURNAL FOR VITAMIN AND NUTRITION RESEARCH, Bd. 57, Nr. 1, 1987, Seiten 37-43, XP009008238 ISSN: 0300-9831

## Beschreibung

Die vorliegende Erfindung betrifft ein Verfahren zum Nachweis von Entzündungsvorgängen. Die vorliegende Erfindung betrifft insbesondere ein Verfahren zum Nachweis von Entzündungsvorgängen im Gewebe von Milchdrüsen im Menschen oder Tieren, insbesondere bei Rindern, sowie einen Testkit zur Durchführung des erfindungsgemäßen Verfahrens. Das Verfahren eignet sich insbesondere zum spezifischen Nachweis von Entzündungen in Milchdrüsen, beispielsweise durch Untersuchung von Milch, wobei das am Entzündungsgeschehen beteiligte Enzym Prostaglandin D Synthase (PGDS) qualitativ oder quantitativ bestimmt wird.

Entzündungen der Milchdrüse des Rindes sind wegen ihres häufigen Vorkommens bei etwa 30 - 40% aller Milchkühe von großer wirtschaftlicher Bedeutung. Zum einen wird durch solche Entzündungen die Milchleistung und die vermarktungsfähige Milchqualität verringert. Zum anderen bedingt ein zu spätes Erkennen und die darauf hin notwendige massive antibiotische Behandlung Wartezeiten, in denen die Milch nicht vermarktet werden kann. Unter Annahme eines Verlustes von 10% des Milchertrages bei 15% der Milchkühe in der Bundesrepublik Deutschland, einer durchschnittlichen Milchleistung von 6 500 kg und einem Kilopreis von 0,45 DM ist mindestens eine wirtschaftliche Einbuße von etwa 210 Mio DM pro Jahr zu kalkulieren.

Alle bisher angewandten diagnostischen Verfahren zum Nachweis von Milchdrüsenentzündungen sind unspezifisch und erlauben keine direkte Einschätzung der Entzündungsvorgänge in der Milchdrüse. Zu diesen bekannten Verfahren zählen beispielsweise die Messung der elektrischen Leitfähigkeit der Milch, die Bestimmung von Zellzahlen oder der Nachweis von Enzymen, die eine bereits erfolgte Gewebeschädigung anzeigen, wie z.B. der Laktatdehydrogenase. Der Nachweis solcher Enzyme ist aber erst relativ spät möglich.

Derzeit verfügbare Schnelltests zur Anwendung vor Ort sind indirekte Tests und beruhen auf dem qualitativen bzw. halbquantitativen Nachweis von Desoxyribonukleinsäure, die aus in der Milch enthaltenen Zellen freigesetzt und visuell beurteilt wird. Eine erhöhte Zellzahl, welche eine Erhöhung der Menge an Desoxyribonukleinsäure bewirkt, wird dabei als Indikator für eine Entzündung in der Milchdrüse angesehen. Während der Test einen Hinweis auf die Zellzahl gibt, ist ein erhöhter Zellgehalt jedoch nicht direkt auf Entzündungsvorgänge zurückzuführen, wie beispielsweise der am Beginn und am Ende der Laktation zu beobachtende stark positive Ausfall zeigt.

Da Entzündungen der Milchdrüse fast ausschließlich auf bakterielle Infektionen zurückgehen, müsste der bakteriologischen Diagnostik ein großer Stellenwert zukommen. Die Praxis zeigt jedoch, dass zwischen den genannten unspezifischen Veränderungen in der Milch und der Bakteriologie selten Korrelationen bestehen. Eine Diagnose vorallem subklinischer Entzündungen ist damit bisher sehr unsicher. Beispiele für funktionelle Proteinindikatoren für subklinische Mastitis sind beispielsweise LDH, NAGase (Zank W., Schlatterer B. Assessment of subacute mammary inflammation by soluble biomarkers in comparison to somatic cell counts in quarter milk samples from dairy cows. J Vet Med A 1998; 45:45-51), Plasminogen und Plasmin (Urech E., Puhan Z., Schällibaum M. Changes in milk protein fraction as affected by subclinical mastitis. J Dairy Sci 1999; 82:945-951) sowie der Marker C5a (Rainard P., Poutrel B. Generation of complement fragment C5a in milk ist variable among cows. J Dariy SCi 1999; 82:2402-2411).

Entzündungen werden durch Entzündungsmediatoren in der Regel nach Initiation durch bakterielle Stoffwechselprodukte ausgelöst. Erste Vorgänge beruhen dabei auf einem lokal erhöhten Phospholipidstoffwechsel, der zu Prostaglandinen und prostaglandin-ähnlichen Stoffwechselprodukten führt. Diese sind zwar im Milchsekret einer entzündeten Milchdrüse nachweisbar, es sind jedoch bisher keine eindeutig einschätzbaren Substanzmuster nachgewiesen oder erkannt worden, was unter anderem mit der Kompliziertheit und dem Aufwand der Analytik zu tun hat (F. Atroshi et al. Inflammation-related changes in cyclic AMP and cyclic GMP in bovine mastitis.Vet Res Commun 1989; 13:427-33; O. O'Sullivan et al. Analysis of prostaglandin D2 metabolites in urin: comparison between enzyme immunoassay and negative ion chemical ionisation gas chromatographymass spectrometry. Prostaglandins Other Lipid Mediat 1999; 57:149-165).

Eine Aufgabe der Erfindung war es deshalb, ein Verfahren und insbesondere einen spezifischen Entzündungsindikator zum Nachweis von Milchdrüsenentzündungen bereitzustellen.

Diese Aufgabe wird gelöst durch ein Verfahren zum Nachweis einer Milchdrüsenentzündung, welches dadurch gekennzeichnet ist, dass man das Enzym Prostaglandin D Synthase (PGDS) oder Teilstücke desselben qualitativ und/oder quantitativ bestimmt. Die Bestimmung erfoigt uevotzugr in Körperflüssigkeiten. Es wurde festgestellt, dass die Prostaglandin D Synthase (PGDS) bei Entzündungsvorgängen im Körper bzw. in Gewebe von Menschen und Tieren ansteigt. PGDS kann bei Entzündungen vermehrt in Körperflüssigkeiten, beispielsweise im Plasma, in der Synovialflüssigkeit, in der Cerebrospinalflüssigkeit, im Urin oder im Ejakulat aufgefunden werden. Die Prostaglandin D Synthase (PGDS) eignet sich somit als

Entzündungsindikator für Entzündungsvorgänge, da die Menge an Enzym bei Entzündungen ansteigt. Die Bestimmung kann auch im Stuhl erfolgen und dient dann zum Nachweis von Entzündungen im Gastrointestinaltrakt, insbesondere im Magen, Pankreas oder Darm.

Die Erfindung betrifft ein Verfahren zum Nachweis einer Milchdrüsenentzündung, welches dadurch gekennzeichnet ist, dass man das Enzym Prostaglandin D Synthase (PGDS) oder Teilstücke desselben qualitativ und/oder quantitativ bestimmt.

Es wurde festgestellt, dass die Prostaglandin D Synthase (PGDS) bei Milchdrüsenentzündungen vermehrt ausgeschieden wird, so dass sich dieses Enzym als Entzündungsindikator zum spezifischen qualitativen oder/und quantitativen Nachweis von Milchdrüsenentzündungen eignet.

Erfindungsgemäß wird somit ein unmittelbar an die Entzündungsvorgänge gekoppelter Indikator von hoher Spezifität bereitgestellt, dessen Nachweis und Quantifizierung auch eine Frühdiagnose von Entzündungen der Milchdrüsen zulässt. Das Indikatorenzym Prostaglandin D Synthase (Zugangsnummer 002853) kommt in mehreren Isoformen vor, die einzein oder zusammen nachgewiesen werden können.

Die Indikatorsubstanz PGDS zum Nachweis einer Milchdrüsenentzündung wurde durch Vergleich des Molkeproteinmusters von Milch von von Milchdrüsenentzündung betroffenen Tieren mit dem von nichtbetroffenen, gesunden Tieren aufgefunden. Dieses Proteinmuster kann beispielsweise durch zweidimensionale Gelelektrophorese (2D-PAGE) erhalten werden, wobei bei Milchproben von Tieren mit Milchdrüsenentzündung 4 zusätzliche Flecken bei einem Molekulargewicht von etwa 26 kDa und einem pH-Bereich von 5,0 bis 6,4 aufgetreten sind. Die aus dem Gel ausgeschnittenen Substanzen zeigten ein sehr ähnliches chromatografisches sowie massenspektrometrisches Muster von chemotryptischen Peptiden. Mittels einer Datenbanksuche konnten die 4 Substanzen als Isoformen der Rinderprostaglandin D Synthase (PGDS) identifiziert werden, wobei in einer der Isoformen ein Cysteinrest zu einer Sulfonsäure oxidiert war.

Es ist somit gelungen, trotz der hochkomplexen Natur der Abwehr- und Entzündungsmechanismen von Milchdrüsen gegen die Invasion von verschiedenen infektiösen Mitteln, einen Marker bereitzustellen, der auf zuverlässige Weise einen qualitativen und/oder quantitativen Nachweis einer Michdrüsenentzündung erlaubt. Auf Grund der großen Menge an Kaseinen in Milch wurde eine Vorfraktionierung von Kuhmilchproteinen im Hinblick auf Molkebestandteile ausgeführt, wodurch eine Polypeptidauflösung erreicht werden konnte, die die Identifizierung des erfindungsgemäßen Markerenzyms PGDS erlaubte.

Überraschenderweise konnte dieses Markermolekül nicht nur im Blut oder innerhalb der Zellen bei einer Milchdrüsenentzündung nachgewiesen werden, sondern tritt bei Milchdrüsenentzündungen auch in Milch in erhöhter Menge auf, so dass ein leichter und einfacher Nachweis einer Milchdrüsenentzündung möglich ist. Weiterhin konnte das Enzym bei Entzündungen in erhöhter Menge in Körperflüssigkeiten nachgewiesen werden, z.B. bei Enzephalitis in erhöhter Menge im Cerebrospinalfluid und bei rheumatoider Arthritis in der Synovialflüssigkeit. Die lipocalin-artige Prostaglandin D Synthase, welche auch als Prostaglandin-H2-D-Isomerase (EC5.3.99.2) bezeichnet wird, ist ein Membran-gebundenes Enzym (Giacomelli S, Leone MG, Grima J, Silvestrini B, Cheng CY. Astrocytes synthesize and secrete prostaglandin D synthase in vitro. Biochim Biophys Acta 1996; 1310:269-276).

Grundsätzlich kann das erfindungsgemäße Verfahren zum Nachweis von Entzündungen bei Menschen oder Tieren eingesetzt werden.

Während das erfindungsgemäße Verfahren bevorzugt zum Nachweis einer Milchdrüsenentzündung bei Menschen oder Tieren eingesetzt werden kann, wird es besonders bevorzugt zum Nachweis von Milchdrüsenentzündungen von Rindern eingesetzt. Bevorzugt bestimmt man das Enzym Prostaglandin D Synthase oder Teilstücke desselben, insbesondere Teilstücke mit einer Länge von mindestens 15 Aminosäuren, mehr bevorzugt von mindestens 30 Aminosäuren und noch mehr bevorzugt von mindestens 50 Aminosäuren, in einer Körperflüssigkeitsprobe oder bevorzugt in einer Milchprobe. Beim Nachweis-beim Menschen wird insbesondere humane Prostaglandin D Synthase als Marker verwendet, beim Nachweis bei Rindern insbesondere bovine Prostaglandin D Synthase.
Die Bestimmung des Markers in einer Milchprobe erlaubt vor Ort eine schnelle und einfache Einschätzung, ob eine Entzündung vorliegt bzw. wie stark eine Entzündung ausgeprägt ist.

Besonders bevorzugt wird die Bestimmung unter Verwendung mindestens eines Antikörpers gegen Prostaglandin D Synthase durchgeführt. Erfindungsgemäß einsetzbare Antikörper können gemäß dem Stand der Technik bekannten Verfahren hergestellt werden. Dem Fachmann sind Verfahren zur Herstellung von monoklonalen bzw. polyklonalen Antikörpern gut bekannt. Zur Herstellung von monoklonalen Antikörpern wird beispielsweise das Antigen, im vorliegenden Fall also das Enzym Prostaglandin D Synthase oder Teilstücke desselben, in üblicher Weise gereinigter Form zur Erzeugung von Antikörpern verwendet. Prinzipiell kann hierzu das Verfahren, welches erstmals von Köhler und Millstein beschrieben wurde, angewandt werden, wobei dem Fachmann auch Abwandlungen und Weiterentwicklungen dieser Verfahren bekannt sind. Die Selektivität der erhaltenen Antikörper kann durch Selektion festgestellt werden.

Es ist auch möglich, ausgehend von Prostaglandin D Synthase oder Teilstücken davon, polyklonale Antikörper nach bekannten Verfahren herzustellen. Solche polyklonalen Antikörper werden bevorzugt eingesetzt, insbesondere polyklonale Antikörper, die alle Isoformen der Prostaglandin D Synthase erkennen.

Besonders bevorzugt wird der Antikörper unter Verwendung der Peptide PGDS1 (Ac-LTSTFLRKDQCETRTLL-NH2), PGDS2 (Ac-FEEDKFLGRWFTSG LAS-NH2) oder PGDS3 (Ac-GPGQDFRMATLYSRSQ-NH2) hergestellt.

Die Bestimmung wird -bevorzugt mittels eines Immuno-Assays, insbesondere mittels eines quantitativen Sandwich-ELISA durchgeführt. In einer bevorzugten Ausführungsform dieses Tests bringt man eine Probe, beispielsweise eine zu untersuchende Milchprobe, mit mindestens zwei verschiedenen Rezeptoren in Kontakt, von denen der erste Rezeptor R1 an eine Festphase immobilisiert vorliegt und mit PGDS bindefähig ist und der zweite Rezeptor R2 in flüssiger Phase vorliegt und ebenfalls mit PGDS bindefähig ist und eine Markierung trägt oder die Bindung an ein detektierbares Molekül mittelt, trennt die feste von der flüssigen Phase und bestimmt die Markierung oder das detektierbare Molekül in der festen Phase. Durch Quantifizierung der bestimmten Menge an Markierung oder detektierbarem Molekül kann die Menge an in der Probe vorhandener PGDS quantifiziert werden.

In einer weiteren bevorzugten Ausführungsform wird das erfindungsgemäße Verfahren als Western-Blot durchgeführt.

Bei Durchführung eines Immuno-Assays kann spezifisch der Gehalt an PGDS in einer Probe, beispielsweise in einer Milchpobe, festgestellt werden, welche als Marker für eine Milchdrüsenentzündung angesehen werden kann. Neben der quantitativen Bestimmung ist auch eine qualitative Bestimmung möglich, um z.B. ein schnelles Resultat vorab zu erhalten. Bevorzugt wird ein cut-off-Wert festgelegt, um negative von positiven Resultaten zu unterscheiden.

In einer bevorzugten Ausführungsform wird zunächst ein an das Enzym PGDS spezifisch bindender Antikörper mittels bekannter Verfahren an eine Festphase immobilisiert. Die zu untersuchende Probe, insbesondere eine Milchprobe wird nach Entrahmung in einer geeigneten Pufferlösung mit dem fixierten Antikörper in Kontakt gebracht und über diesen an die Festphase gebunden. Nach Waschen des so erhaltenen immobilisierten Antikörper-Enzym-Komplexes wird dann ein weiterer mit einem Marker gekoppelter sekundärer Antikörper zugefügt, der an ein anderes Epitop der PGDS bindet und die Menge des im System verbleibenden Markers bestimmt. Die Menge des gebundenen Markers ist direkt proportional zur Menge an PGDS in der Probe. Bevorzugt wird das erfindungsgemäße Verfahren parallel mit Referenzmaterial mit unterschiedlichen Konzentrationen von PGDS in Körperflüssigkeitsproben, insbesondere in Milchproben durchgeführt, um ein hohes Maß an Analysensicherheit und Genauigkeit bereit zu stellen.

Mit dem erfindungsgemäßen Verfahren können sowohl eine beginnende als auch eine schon bestehende Milchdrüsenentzündung nachgewiesen und quantifiziert werden. Weiterhin ist das Verfahren spezifisch und leicht durchführbar. Das erfindungsgemäße Verfahren eignet sich darüber hinaus nicht nur zur Durchführung in einem Labor, beispielsweise als quantitatives Testverfahren, sondern kann auch als Schnelltestvariante ausgestaltet sein, die es erlaubt, vor Ort die Lokalisation und Schwere einer Entzündung einzuschätzen.

Neben der Durchführung des erfindungsgemäßen Verfahrens in Form eines Immuno-Assays und insbesondere eines ELISA oder eines Western-Blots, ist der Nachweis von PGDS, beispielsweise in Milch, auch mit Hilfe anderer Nachweisverfahren möglich, wobei insbesondere der Nachweis mit Hilfe von Massenspektroskopie, insbesondere MALDI-TOF (matrixunterstützte Laserdesorption/lonisations-Flugzeitmassenspektrometrie) oder mittels Gelelektrophorese, insbesondere zweidimensionaler Gelelektrophorese, bevorzugt ist. Daneben kann die Analytkonzentration prinizipiell auch mittels Biosensoren bestimmt werden, wie z.B. amperometrische Sensoren, potentiometrische, piezoelektrische, thermometrische oder photometrische Sensoren oder auch mittels Halbleiterelektroden, wie Feldeffekt-Transistoren (FIT), chemosensitiven Feldeffekt-Transistoren (CHEMFET), Suspended-gate-Feldeffekt-Transistoren (SGFET) oder ionensensitiven Feldeffekt-Transistoren. Solche Biosensoren sind beispielsweise bei E. A. H. Hall und G. Hummel in "Biosensoren", Springer-Verlag Heidelberg, Deutschland, 1995, beschrieben. Weiterhin kann der Nachweis mittels Kandelaber-Technologie von IBM, lnc. durchgeführt werden. Eine besonders vorteilhafte Verfahrensführung bezüglich Sensitivität, dynamischer Messbereich, Analysenkinetik und Formatflexibilität lässt sich durch die Verwendung der Elektrochemielumineszenz-Technologie erreichen. Unter Elektrochemilumineszenz versteht man einen Prozess, bei welchem Licht freigesetzt wird. Die Lichtfreisetzung wird dadurch induziert, indem ein elektrisches Potenzial an eine Elektrode angelegt wird, welche eine cyclische Redox-Reaktion eines Ruthenium-Metall-lons vermittelt (Bruno, G. (1997) Rec. Rp. Seiten 175-179; Williams R. (1996), Amer. Biotech., Seite 26). Eine ähnliche geeignete Technologie ist die TRACE-Technologie der Firma CIS, Deutschland.

Das erfindungsgemäße Verfahren kann durchgeführt werden mittels eines Testkits zum Nachweis oder/und zur Diagnose einer Milchdrüsenentzündung, welcher mindestens einen mit Prostaglandin D Synthase bindenden Rezeptor enthält, wobei der Testkit insbesondere für die Durchführung des erfindungsgemäßen Verfahrens ausgestaltet ist. Der Testkit ist insbesondere zum Nachweis bei Rindern vorgesehen, kann aber auch zum Nachweis bei anderen Tieren oder beim Menschen vorgesehen sein.

Der Antikörper ist bevorzugt ein poiyktonater Antikörper, der an mehrere, bevorzugt alle Isoformen der Prostaglandin D Synthase bindet.

Der an das Enzym PGDS bindende Rezeptor vermittelt vorzugsweise die Bindung an eine feste Phase, so dass eine Trennung von flüssiger Phase, enthaltend die Probe, insbesondere eine Milchprobe, und fester Phase mit der daran über einen Rezeptor gebundener PGDS ermöglicht wird. Diese Festphase ist in einer bevorzugten Ausgestaltung ebenfalls Bestandteil des Testkits. Die zu untersuchende Probe wird dann in einer geeigneten Pufferlösung mit dem fixierten oder fixierbaren Rezeptor, insbesondere Antikörper, in Kontakt gebracht und mittels des Rezeptors an die Festphase gebunden. Bei der Verwendung von Milch als Probe wird bevorzugt vor diesem Schritt eine Entrahmung durchgeführt.

Nach Waschen des erhaltenen immoblisierten Antikörper-PGDS-Komplexes wird dann ein weiterer markierter oder an ein detektierbares Molekül bindefähiger, z.B. mit Biotin versehener sekundärer Antikörper zugesetzt, der vozugsweise an ein anderes Epitop der PGDS bindet. Anschließend kann die Markierung bestimmt werden, z.B. mit Hilfe von Streptavidin-Peroxidase. Die Menge an gebundenem Marker ist direkt proportional zur Menge an PGDS in der Probe. Zweckmäßigerweise enthält der Testkit Referenzmaterial bekannten Gehalts und insbesondere mit unterschiedlichen Konzentrationen von PGDS zur quantitativen Bestimmung und zur Sicherung der Analysenqualität.

Neben der bevorzugten Ausgestaltung als Festphasen-Sandwich-Assay kann das erfindungsgemäße Verfahren auch durch andere Nachweismethoden durchgeführt werden. Insbesondere können alle Arten von immunologischen Nachweisverfahren, welche mit Hilfe von Antikörpern durchgeführt werden, erfindungsgemäß eingesetzt werden. So kann das erfindungsgemäße Verfahren beispielsweise als Sandwich-, ELISA-, Schwingquarz-, Mikrowaage- oder Elektrochemolumineszenztest durchgeführt werden und enthält bevorzugt die dafür notwendigen Reagenzien und gegebenenfalls auch Vorrichtungen.

Für eine einfache Handhabung kann der Test in Form eines Teststreifens konzipiert werden, auf dem beispielsweise in unterschiedlichen Zonen die benötigten Antikörper, entweder in löslicher Form oder Festphasen fixiert, angeordnet sind. Die Probe bzw. der Flüssigkeitsanteil der Probe oder ein Extrakt davon können dann den Teststreifen durchwandern und an der Detektionsstelle ein Signal-liefern, wenn PGDS-in-der Probe vorhanden ist.

In einer weiteren bevorzugten Ausführungsform ist der Test als Schnelltest in Form eines Membrandiffussionstests ausgestaltet, z.B. als immunchromatografischer Lateral Flow Test oder ähnlicher Test.

Im Rahmen der vorliegenden Erfindung bezeichnet der Ausdruck "Rezeptor" bzw. "Antikörper" auch solche Teile oder Fragmente von Rezeptoren bzw. Antikörpern, welche die notwendige Bindung an PGDS noch vermitteln. Es ist dabei auch möglich, an Stelle eines einzelnen Antikörpers ein Konjugat, beispielsweise aus zwei Antikörpern, einzusetzen. Insbesondere kann beispielsweise als sekundärer Rezeptor ein mit PGDS bindefähiger Antikörper verwendet werden und der Nachweis über einen weiteren markierten Antikörper geführt werden, welcher gegen den Fc-Teil des sekundären Antikörpers gerichtet ist und eine Markierung trägt oder wiederum an ein detektierbares Molekül gekoppelt ist. Eine solche Konjugatbildung aus zwei Antikörpern soll im Rahmen der vorliegenden Erfindung mit umfasst sein, wenn der sekundäre Antikörper so definiert ist, dass er die Bindung an ein detektierbares Molekül vermittelt. Analoges gilt für die Bindung des ersten Antikörpers an die feste Phase. Auch diese Bindung kann über Festphasen-gekoppelte Antikörper erfolgen, welche an den Fc-Teil des ersten Antikörpers binden.

Im Rahmen der vorliegenden Erfindung ist es bevorzugt, dass der verwendbare Testkit als Rezeptoren Antikörper oder spezifisch bindefähige Antikörperfragmente enthält. Es ist weiterhin bevorzugt, dass der erste Antikörper an eine feste Phase gekoppelt vorliegt, während der sekundäre Antikörper bevorzugt ein löslicher, markierter Antikörper oder ein löslicher Antikörper ist, der an ein Enzym gebunden ist, welches wiederum über eine Nachweisreaktion bestimmbar ist. Die Markierung kann so ausgestaltet sein, dass sie ohne weitere Zugaben von Substanzen erkennbar ist, wie z.B. eine Goldmarkierung oder eine Fluoreszenzmarkierung, aber auch so ausgestaltet sein, dass eine Zugabe weiterer Reagenzien die Bestimmung ermöglicht. Beispielsweise kann der Nachweis durch Markierung mit Enzymen und Zugabe des Enzymsubstrats erfolgen.

Ein weiterer Gegenstand der vorliegenden Erfindung ist die Verwendung eines Antikörpers gegen Prostaglandin D Synthase zur Herstellung eines Mittels zum Nachweis von Milchdrüsenentzündungen. Erfindungsgemäß wurde festgestellt, dass Antikörper gegen Prostaglandin D Synthase spezifische Marker für Milchdrüsenentzündungen sind, so dass sie sowohl zum qualitativen als auch zum quantitativen Nachweis solcher Entzündungen eingesetzt werden können.

Bevorzugt wird im erfindungsgemäßen Verfahren ein monoklonaler Antikörper eingesetzt, welcher spezifisch das Enzym Prostaglandin D Synthase (PGDS) bindet.

Die Antikörper sind mit Hilfe von an sich bekannten Methoden erhältlich. Hierbei wird zunächst das Enzym Prostaglandin D Synthse (PGDS) isoliert und gegebenenfalls gereinigt. Danach kann ein Versuchstier mit der so erhaltenen Prostaglandin D Synthase bzw. mit Bruchstücken davon, welche die entsprechenden Epitope aufweisen, immunisiert und die gebildeten Antikörper isoliert werden. Bruchstücke, welche zur Immunisierung verwendet werden, können z.B. aus einer Protease-Verdauung der gereinigten PGDS stammen oder aus synthetischen Teilpeptiden desselben bestehen. Die Herstellung solcher Teilpeptide ist dem Fachmann an sich bekannt. Es können dabei aus der Gesamtsequenz z.B. mit Hilfe von Computerprogrammen Teilsequenen ausgewählt werden, welche entsprechende Epitope enthalten. Diese Sequenzen werden dann auf ihre Verwendbarkeit zur Herstellung spezifischer Antikörper getestet.

Vorzugsweise werden die monoklonalen Antikörper nach der Methode von Köhler, Milstein (Nature 256, 495-497 (1975)) erzeugt. Dabei werden beispielsweise BALB/c-Mäuse mit isolierter PGDS immunisiert und die Milzzellen dieser Tiere mit einer Myelomazelllinie, beispielsweise PA I, fusioniert. Die sezernierten Antikörper werden beispielsweise in ELISA oder RIA auf ihre Spezifität getestet und isoliert.

Des Weiteren können Aptamere eingesetzt werden, welche spezifisch an PGDS binden. Aptamere sind Oligonukleotidsequenzen, welche spezifische Bindeeigenschaften aufweisen. Die Aptamere können beispielsweise nach den in US 5,270,163 oder in Sumedha, Clin. Chem. 45 (1999) 1628-1650 beschriebenen Methoden hergestellt bzw. identifiziert werden.

Der Erfindung wird durch die beigefügten Figuren und die folgenden Beispiele weiter erläutert.

### Figur 1:

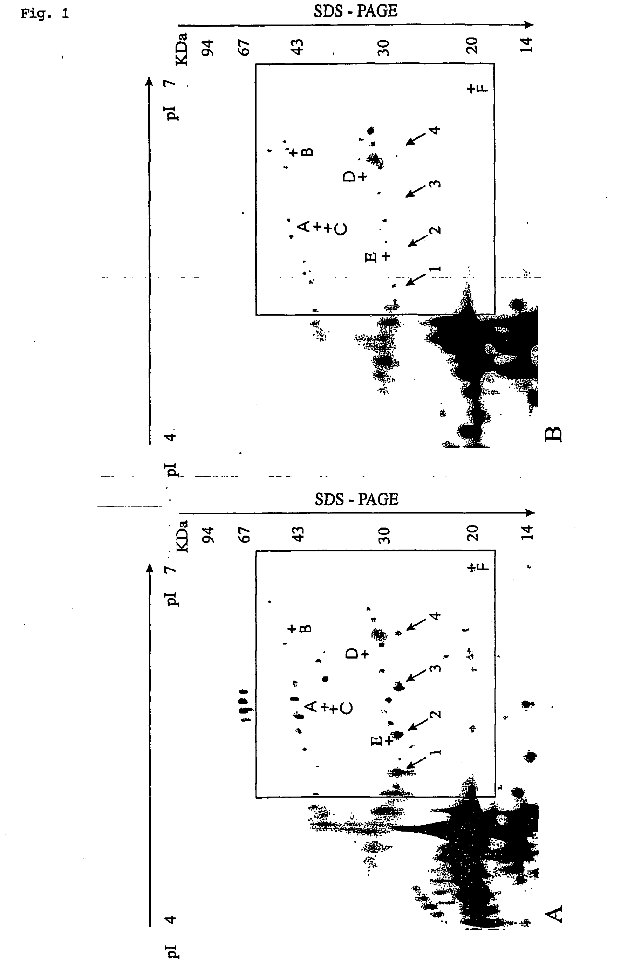

Vergleich von Molkeproteinmustern von entzündeten (Figur 1 A) und unveränderten (Figur 1 B) Milchdrüsenvierteln mit SDS-2D-PAGE

Äquivalente Mengen (6 mg) von lyophilisierten Proteinproben wurden auf pH 4-7 lineare IPG-Streifen geladen und fokussiert, gefolgt von

Elektrophorese auf einem SDS-Gel mit konstanter Acrylamidkonzentration (12% T, 2,67% C). Pfeile zeigen die identifizierten Proteinspezies, die in dem unveränderten Euterviertel nicht vorhanden sind. Die mit Buchstaben bezeichneten Flecken stellen identifizierte Proteine dar, die üblicherweise in jeder Milch gefunden werden: A:IgG1; B:Rinderserumalbumin-Fragment; C:Rinderserumalbumin-Fragment; D:Rinderserumalbumin-Fragment; E:*κ-*Casein; F:EPI (sekretorisches Protein).

### Figur 2:

Umkehrphasen-HPLC-Separation der chymotryptischen Peptide, die durch einen Verdau der Proteine der Flecken 2 und 3 im Gel erhalten wurden.

### Figur 3:

MALDI-TOF-Spektren im negativen lonenmodus der chymotryptischen Peptide, die durch Verdau der Proteine in der Flecken 2 und 3 im Gel erhalten wurden. Die Spektren sind fast identisch außer-der durch Pfeile markierten Peptide. In Fleck 3 können die Peptide bei 1.085 und 1.198 Da den Resten 68 bis 77 und 67 bis 77 der PGD-S-Sequenz zugeordnet werden. Die Massen der entsprechenden Peptide im Fleck 2 sind 9 Da niedriger.

### Figur 4:

PGDS-Sequenz. Die beobachteten chymotryptischen Peptide sind unterstrichen. Peptide, die im Fleck 2 und 3 unterschiedlich sind, sind fettgedruckt.

### Figur 5:

Western-Blots von Milchsekreten aus unterschiedlich veränderten Eutervierteln.

### Beispiele

### Beispiel 1

### Materialien und Methoden

Es wurden 7 Kühe untersucht, ausgewählt auf Basis einer bakteriologischen Untersuchung, hinsichtlich der somatischen Zellzahl (SCC) und der Aktivität von Lactatdehydrogenase (LDH). Vormilchproben von infizierten und nichtinfizierten Vierteln des gleichen Euters wurden direkt in 50 ml Behälter abgefüllt, die eine Kombination der zwei Proteinaseinhibitoren PMSF (Phenylmethylsulfonylfluorid, 2 mM) und APMSF ((4-Amidinophenyl)-2 Methansulfonylfluoridhydrochlorid, 20 µM), beide von Roche Diagnostics Mannheim, enthielten. Für die Bestimmung der Zellzahlen und der Aktivität von LDH wurden nicht-gespikte Vormilchproben verwendet.

### 1.1 Analyse und Herstellung von Molkefraktionen

SSC wurde mit einem Fossomatic 360 (Foss Electric, Hillerfod, Denmark) gemessen. Die LDH-Aktivität wurde in entrahmter Milch mit einem Beckman Synchron CX5 CE Autoanalysator gemessen (Zank W, Schlatterer B. Assessment of subacute mammary inflammation by soluble biomarkers in comparison to somatic cell counts in quarter milk samples from dairy cows. J. Vet. Med. A 1998; 454:45-51). Die Ergebnisse wurden in LDH-Einheiten (µmol/sec I) ausgedrückt. Die Molkeproteinfraktionen wurden gemäß dem Verfahren von Molloy MP, Herbert BR, Yan JX, Williams KL, Gooley AA. (Identification of wallaby milk proteins separated by twodimensional electrophoresis, using amino acid analysis and sequence tagging. Electrophoresis 1997; 18:1073-1078) hergestellt. Kurz, eine Milchfettabtrennung, wurde durch Zentrifugation bei 5000 g, 4°C für 10 min durchgeführt. Casein wurde durch Ansäuerung von entrahmter Milch auf einen pH von 4,6 mit 4 M Essigsäure prezipitiert und durch Zentrifugation bei 5000 g, 4°C und durch Filtration, zunächst durch einen Papierfilter und dann durch eine Polyvinyliden-difluorid-Membran (PVDF, 0,2 µm) entfernt. Das Filtrat wurde über Nacht bei 4°C gegen doppelt destilliertes Wasser dialysiert. Nicht aufgelöstes Material wurde durch Zentrifugation entfernt. Eine Proteinbestimmung wurde gemäß Bradford MH. (A rapid and sensitive method for quantitation of microgram quantities of protein utilizing the principle of protein-dye binding. Anal Biochem 1976; 205:22-26) durchgeführt unter Verwendung eines Protein-Assays von Bio-Rad (Hercules, USA). Aliquots wurden lyophilisiert und bei -70°C gelagert.

### 1.2 Zweidimensionale Polyacrylamid-Gelelektrophorese (2D-PAGE)

### 1.2.1 Erste Dimension: Isoelektrisches Fokussieren

6 mg lyophilisierter Proteine der Molkefraktionen wurden in 350 µl Rehydratationslösung (8 M Harnstoff, 2% CHAPS, 0,3% Dithioerythritol (DDT), 2% IPG-Puffer) gelöst. Immobiline^{™} Dry Strips, pH 4-7, 18 cm, wurden auf einer Multiphor II-Vorrichtung (Amersham Pharmacia Biotech, Uppsala, Schweden) eingesetzt. Das Fokussieren wurde bei 500 V gestartet. Die Spannung wurde schrittweise innerhalb einer Stunde auf 3500 V erhöht und das Fokussieren wurde bei 2 mA und 10 W für 7,5 h fortgesetzt.

### 1.2.2 Zweite Dimension: SDS-Polyacrylamid-Gelelektrophorese

Vor der zweiten Dimension wurden die IPG-Streifen zunächst für 15 min in 10 ml aus 50 mM Tris-HCl, pH 8,8, 6 M Harnstoff, 30% (vol/vol) Glycerin, 2% (Gew/vol) SDS, einer Spur Bromphenol blau und 65 mM Dithioerythritol (DDT) äquilibriert und dann für weitere 15 min im gleichen Puffer, wobei DTT durch 260 mM Jodacetamid ersetzt wurde. Auf einem SDS-Gel mit konstanter Acrylamidkonzentration (12% T, 2,67% C, 200x205x1,5 mm) wurden die äquilibrierten IPG-Streifen in 0,5% Agarose auf der kathodischen Seite einbettet unter Verwendung der horizontalen Protean® II-Vorrichtung (Bio-Rad, Richmond, USA). Die Elektrophoresebedingungen waren 150 V, 15mA/gel, 10 W bei 12°C über Nacht, bis der Bromphenol-Nachweisfarbstoff am anodischen Ende erschien. Die Gele wurden mit Coomassie blau R 350 gefärbt, bis die erforderliche Färbungsintensität erhalten war. Ein Entfärben der Gele wurde durch mehrmaliges Wechseln einer Eritfärbelösung (25% Ethanol, 8% Essigsäure) durchgeführt.

### 1.3 Identifizierung und Analyse der Gel-aufgelösten Proteine

### 1.3.1 Proteolytischer Verdau der Proteine im Gel und Extraktion der Peptide

Die gefärbten Proteinflecken-wurden ausgeschnitten und 2-6 h in einem Gemisch aus 40% (vol/vol) Acetonitril und 60% (vol/vol) 50 mM NH₄HCO₃ entfärbt. Nach Trocknung durch Vakuumzentrifugation wurden sie mit einer Chymotrypsinlösung (30 ng/µl in 50 mM NH₄HCO₃) getränkt. Der Verdau wurde über Nacht bei Raumtemperatur durchgeführt. Die Peptide wurden durch Zugabe von 30 µl 0,1% Trifluoressigsäure (TFA) gefolgt von 50 µl Acetonitril (CH₃CN) nach 10 min extrahiert. Der Überstand wurde entfernt und die Vorgehensweise einmal wiederholt. Die kombinierten Überstände wurden lyophilisiert.

### 1.3.2 RP-HPLC (Umkehrphasen-Hochdruckflüssigkeitschromatographie) Trennung der chymotryptischen Peptide

Die durch enzymatischen Verdau (Chymotrypsin) gebildeten Peptide wurden durch RP-HPLC (SMART system, Pharmacia, Uppsala, Schweden) auf einer Pharmacia C2/C18 SC2.1 /10 - Säule unter Verwendung eines linearen (0-50% in 40 min) Acetonitrilgradienten in 0,1 % TFA getrennt. Die Peptide wurden bei einer Wellenlänge von 214 nm detektiert.

### 1.3.3 MALDI-TOF-Massenspektrometrie

MALDI-TOF (Matrix-unterstützte Laserdesorption/lonisation-Flugzeitmassenspektrometrie) Massenspektren wurden auf einen Reflex II MALDI-TOF Instrument (Bruker-Daltonik, Bremen, Deutschland) aufgenommen. Alle Spektren wurden im Reflektormodus mit α-Cyano-4-Hydroxyzimtsäure (15 mg/ml in 70% Acetonitril) als Matrix aufgenommen. Zur massenspektrometrischen Analyse wurde das Peptidgemisch in 10 µl eines 3/7 Gemisches (vol/vol) Acetonitril und 2% wässriger TFA gelöst. Einzelne durch RP-HPLC gereinigte Peptide wurden einer weiteren Analyse durch Aufzeichnen der Post-Source-Decay-Spektren unterzogen.

### 1.4 Datenbanksuche

Die Datenbanksuche unter Verwendung der proteolytischen Peptidmassen wurde mit dem Peptid Search Programm entwickelt bei der EMBL Protein & Peptide Group (http://www.mann.embl-heidelberg.de oder http://www.peptsearch.protana.com) durchgeführt. PeptidSearch verwendet eine nicht-redundante Proteindatenbank, welche gegenwärtig mehr als 465.000 Eintragungen enthält. Die Identifizierung der Proteine basiert auf dem Vergleich des Satzes an Peptidmassen, die experimentell von den isolierten Proteinen abgeleitet wurden, mit den enzymatisch abgeleiteten theoretischen Massen der enzymatisch abgeleiteten Peptide von allen Proteinsequenzen in der Datenbank.

### 2. Ergebnissse

### 2.1 Entzündungsmarker

Auswahlkriterien für subklinisch entzündete Milchdrüsenviertel waren LDH-Aktivitäten von mehr als 2 µmol/sec I, somatische Zellzahlen höher als 250.000 ml⁻¹ und, falls bestimmt, positive bakteriologische Ergebnisse. Für Vergleichszwecke wurden nur veränderte und nichtveränderte Viertel des gleichen Euters verwendet. Die untersuchten Euterviertel der ausgewählten Kühe sind Tabelle 1 gezeigt.

**Tabelle 1: Einzelne Kühe mit entzündeten (1) und unveränderten (2) Milchdrüsenvierteln**

| | | | | |
|---|---|---|---|---|
| **KUH** | **VIERTEL** | **LDH-EINHEITEN µmol/sec I** | **SCC** | **MIKROBIOLOGIE** |
| 1 | 1 | 12,23 | 9.911.000 | |
| | | | | n.d. |
| | 2 | 1,21 | 30.000 | |
| 2 | 1 | 19,19 | 5.784.000 | |
| | | | | n.d. |
| | 2 | 4,22 | 203.000 | |
| 3 | 1 | 16, 50 | 5.010.000 | |
| | | | | n.d. |
| | 2 | 1,67 | 29.000 | |
| 4 | 1 | 2,53 | 984.000 | |
| | | | | Streptococcus spp. |
| | 2 | 1,06 | 29.000 | |
| 5 | 1 | 17,42 | 4.048.000 | |
| | | | | Staphylococcus spp. |
| | 2 | 1,58 | 162.000 | |
| 6 | 1 | 28,11 | 7.697.000 | |
| | | | | Streptococcus spp. |
| | 2 | 2,86 | 50.000 | |
| 7 | 1 | 5,21 | 1.436.000 | |
| | | | | Staphylococcus spp. |
| | 2 | 0,99 | 71.000 | |

| | | | | |
|---|---|---|---|---|
| n.d.: nicht bestimmt | | | | |

### 2.2 SDS-2D-PAGE Visualisierung der Molkefraktionen

Der deutlichste Unterschied in den 2D-PAGE-Mustern ist das Erscheinen von 4 Flecken in den Molkefraktionen, die von entzündeten Vierteln erhalten wurden, verglichen mit solchen von den jeweiligen internen Kontrollen. Diese Flecken sind bei einem Molekulargewicht von 26 kDa über einen pI-Bereich von 5,2 bis 6,2 angeordnet und in Figur 1 mit den Zahlen 1-4 markiert.

### 2.3 Identifizierung der Proteine innerhalb der Flecken

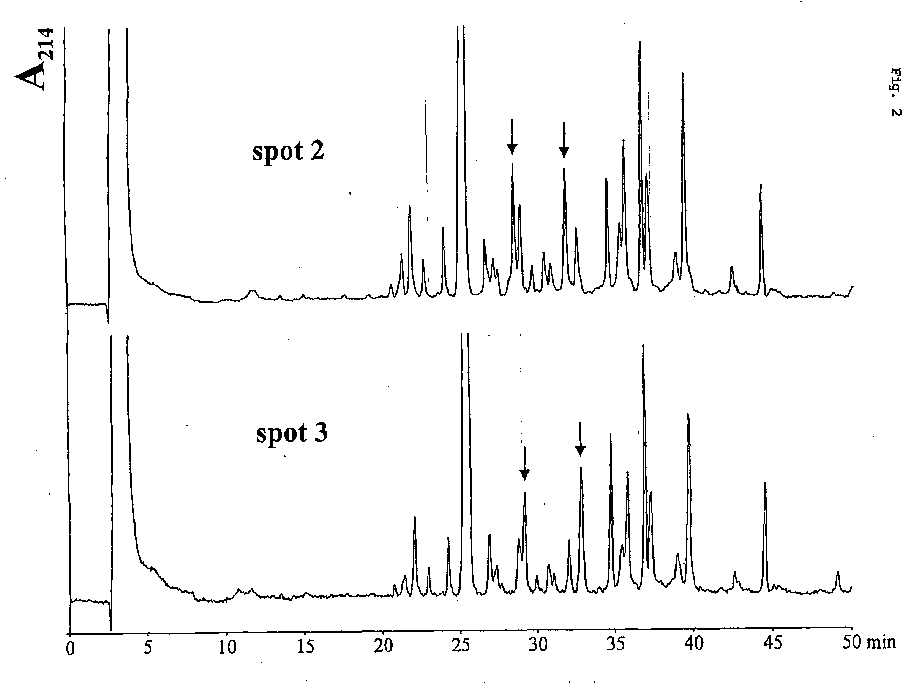

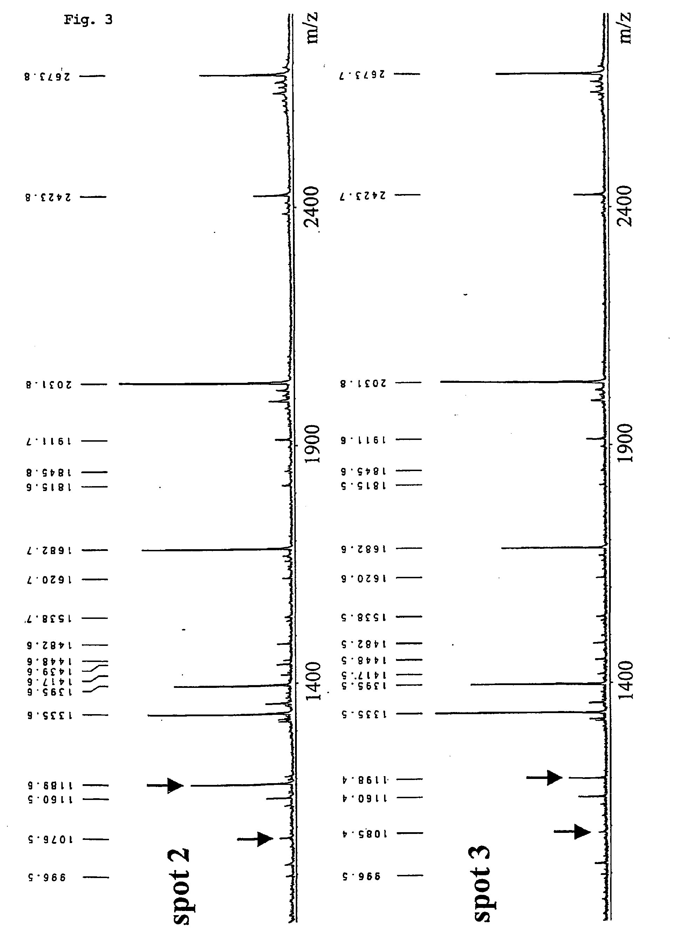

Die Flecken 1-4, die aus den 2D-Gelen ausgeschnitten wurden, ergaben sehr ähnliche chromatografische sowie massenspektrometrische Muster von chymotryptischen Peptiden (die für den Flecken 2 und 3 erhaltenen Muster sind in den Figuren 2 und 3 gezeigt). Eine Datenbanksuche basierend auf den festgestellten Peptidmassen ermöglichte es, die Proteine zweifelsfrei als Lipocalin-artige Prostaglandin D Synthase (Zugangsnummer 002853) zu identifizieren. Weiterhin erlaubten es die massenspektrometrischen Daten festzustellen, dass das reife Protein beim Rest 39 der Datenbanksequenz beginnt.

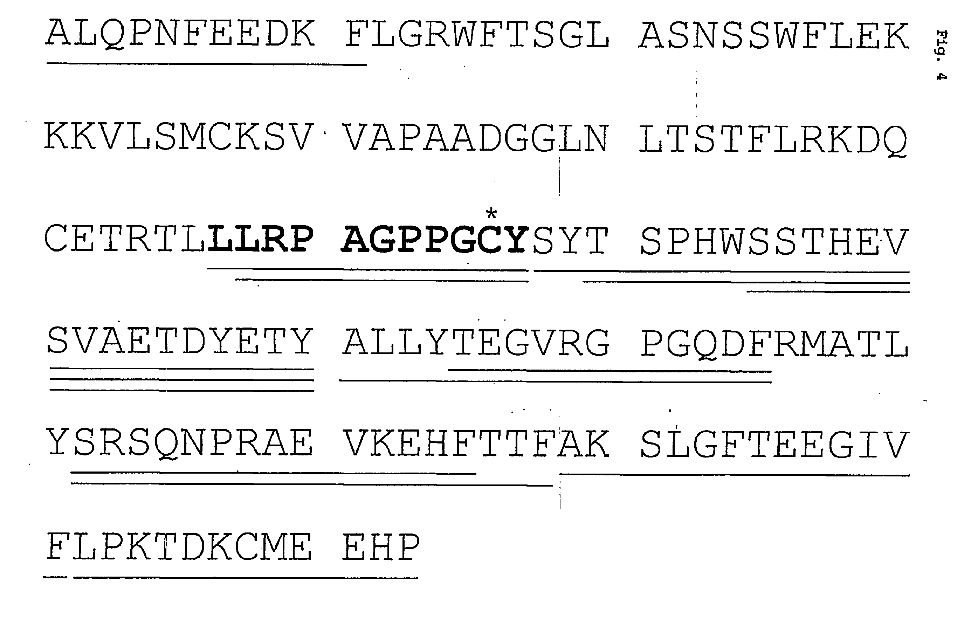

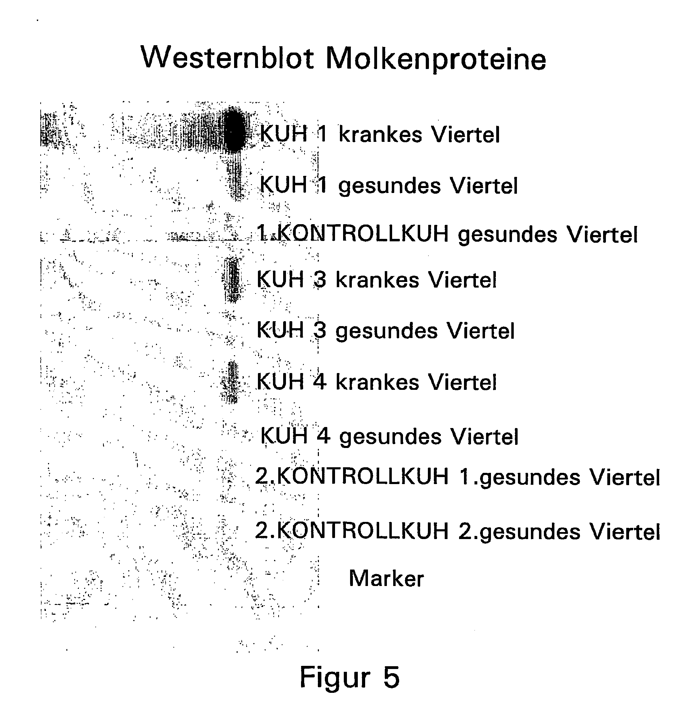

Beim Vergleich der aus den Flecken 2 und 3 erhaltenen Peptidmuster können zwei Unterschiede detektiert werden, wie durch die Pfeile in Figur 2 und Figur 3 angedeutet. Für den Fleck 3 können die entsprechenden Peptide den Sequenzabschnitten LLRPAGPPGCY und LRPAGPPGCY zugeordnet werden (siehe Figur 4). Die entsprechenden Peptide von Fleck 2 zeigen eine Massendifferenz von -9 Da. Post-Source-Decay-Spektren der 2 durch RP-HPLC isolierten Peptide ermöglichte es, die Modifikation einem Cysteinrest in Position 76 des reifen Proteins zuzuordnen. Die Massendifferenz von
-9 Da entspricht der Differenz zwischen einem mit einem Jodacetamid alkylierten Cystein und einem zu Sulfonsäure oxidierten Cystein. Dies stimmt auch mit der Beobachtung überein, dass der isoelektrische Punkt des modifizierten Proteins zu einem sauren Wert verschoben ist. Die Modifikation durch Jodacetamid (Fleck Nr. 3) tritt während den Gelelektrophorese-Bedingungen im zweiten Äquilibrierungsschritt auf, wohingegen das oxidierte Cystein (Fleck 2) bereits vor der 2D-Gelelektrophorese als Sulfonsäurederivat vorliegt. Da die Einführung einer Sulfonsäuregruppe einen starken Einfluss auf den isoelektrischen Punkt eines Proteins hat, können die unterschiedlichen oxidierten Cysteine die Beobachtung von mehreren Gelflecken mit sehr ähnlichen Molekulargewichten aber unterschiedlichen pl erklären. Die unterschiedlichen Spots können aber auch durch eine posttranslationale Modifikation, wie etwa ein heterogenes Glycosilierungsmuster hervorgerufen werden.

### Beispiel 2:

### Western-Blot

Es wurden Western-Blots von Milchsekreten aus unterschiedlich veränderten Eutervierteln durchgeführt. Die für den Blot verwendeten polyklonalen Antikörper wurden gegen Prostaglandin D Synthase aus Rindersperma erzeugt.

Für den Vergleich wurden jeweils Paare aus der gleichen Kuh herangezogen. Bei den erkrankten Milchdrüsen ist eine deutliche Färbung an der entsprechenden Position, an der der polyklonale Antikörper aufgetragen war, zu erkennen. Bei Kuh 1 war das "gesunde" Viertel ebenfalls auf dem Weg zu einer Entzündung, was zeigt, dass mit dem erfindungsgemäßen Verfahren Milchdrüsenentzündungen bereits in einem sehr frühen Stadium erkannt werden können. Um eine sichere Diskriminierung zwischen positiven (d.h. entzündeten) und negativen (d.h. gesunden) Resultaten zu erhalten, kann ein cut-off-Wert festgelegt werden oder/und gleichzeitig Referenzproben mit vermessen werden.

## Patentansprüche

1. In-vitro Verfahren zum Nachweis einer Entzündung,
**dadurch gekennzeichnet,**
**dass** man das Enzym Prostaglandin D Synthase (PGDS) oder Teilstücke desselben qualitativ und/oder quantitativ bestimmt und
**dass** es sich bei der nachzuweisenden Entzündung um Milchdrüsenentzündung handelt.

2. Verfahren nach Anspruch 1 zum Nachweis von Entzündungsvorgängen in Menschen oder Tieren.

3. Verfahren nach Anspruch 1 oder 2 zum Nachweis von Milchdrüsenentzündungen von Rindern.

4. Verfahren nach Anspruch 1 oder 3,
**dadurch gekennzeichnet,**
**dass** man das Enzym Prostaglandin D Synthase (PGDS) oder Teilstücke desselben in einer Körperflüssigkeitsprobe, in einer Stuhlprobe oder in einer Milchprobe bestimmt.

5. Verfahren nach Anspruch 4,
**dadurch gekennzeichnet,**
**dass** die Körperflüssigkeitsprobe ausgewählt ist aus Plasma, und Urin.

6. Verfahren nach einem der vorhergehenden Ansprüche,
**dadurch gekennzeichnet,**
**dass** man die Bestimmung unter Verwendung mindestens eines Aptamers oder/und Antikörpers gegen Prostaglandin D Synthase durchführt.

7. Verfahren nach Anspruch 6,
**dadurch gekennzeichnet,**
**dass** man einen polyklonalen oder/und monoklonalen Antikörper verwendet.

8. Verfahren nach einen der Ansprüche 6 oder 7,
**dadurch gekennzeichnet,**
**dass** man einen Antikörper verwendet, der unter Verwendung mindestens eines der Peptide PGDS1, PGDS2 oder/und PGDS3 erhältlich ist.

9. Verfahren nach einem der vorhergehenden Ansprüche,
**dadurch gekennzeichnet,**
**dass** man die Bestimmung mittels eines Immuno-Assays, insbesondere eines quantitativen Sandwich-ELISA oder eines Western-Blots durchführt.

10. Verfahren nach einem der vorhergehenden Ansprüche,
**dadurch gekennzeichnet,**
**dass** der Nachweis mit Hilfe von Massenspektroskopie, insbesondere MALDI-TOF oder mittels Gelelektrophorese, insbesondere zweidimensionaler Gelelektrophorese erfolgt.

11. Verwendung eines Aptamers oder eines Antikörpers gegen Prostaglandin D Synthase (PGDS) zur Herstellung eines Mittels zum Nachweis von Milchdrüsenentzündungen.

## Claims

1. In-vitro method for detection of inflammation,
**characterized**
**in that** the enzyme prostaglandin D synthase (PGDS)
or parts of it is or are determined qualitatively and/or quantitatively, and in that the inflammation to be detected is mammary gland inflammation.

2. Method according to Claim 1 for detection of inflammation processes in humans or animals.

3. Method according to Claim 1 or 2, for detection of mammary gland inflammation in cattle.

4. Method according to one of Claims 1 to 3,
**characterized**
**in that** the enzyme prostaglandin D synthase (PGDS)
or parts of it is or are determined in a bodily fluid sample, in a stool sample, or in a milk sample.

5. Method according to Claim 4,
**characterized**
**in that** the bodily fluid sample is chosen from plasma and urine.

6. Method according to one of the preceding claims,
**characterized**
**in that** the determination is made using at least one aptamer or/and antibody against prostaglandin D synthase.

7. Method according to Claim 6,
**characterized**
**in that** a polyclonal and/or monoclonal antibody is used.

8. Method according to one of claims 6 or 7,
**characterized**
**in that** an antibody is used which can be obtained using at least one of the peptides PGDS1, PGDS2 and/or PGDS3.

9. Method according to one of the preceding claims,
**characterized**
**in that** the determination is made by means of a immunoassay, in particular a quantitative sandwich ELISA or a Western Blot.

10. Method according to one of the preceding claims,
**characterized**
**in that** the detection is made with the aid of mass spectroscopy, in particular MALDI-TOF or by means of gel electrophoresis, in particular two-dimensional gel electrophoresis.

11. Use of an aptamer or of an antibody against prostaglandin D synthase (PGDS) in order to produce a means for detection of mammary gland inflammation.

## Revendications

1. Procédé *in vitro* de détection d'une inflammation, **caractérisé en ce que** l'on détermine de manière qualitative et/ou quantitative l'enzyme prostaglandine-D-synthase (PGDS) ou des fragments de celle-ci et **en ce que** l'inflammation à détecter est une inflammation des glandes mammaires.

2. Procédé selon la revendication 1 pour la détection de processus inflammatoires chez l'homme ou l'animal.

3. Procédé selon la revendication 1 ou 2 pour la détection d'inflammations des glandes mammaires chez les bovins.

4. Procédé selon la revendication 1 ou 3, **caractérisé en ce que** l'on détermine l'enzyme prostaglandine-D-synthase (PGDS) ou des fragments de celle-ci dans un échantillon de liquide corporel, dans un échantillon de selles ou dans un échantillon de lait.

5. Procédé selon la revendication 4, **caractérisé en ce que** l'échantillon de liquide corporel est choisi parmi le plasma et l'urine.

6. Procédé selon l'une des revendications précédentes, **caractérisé en ce que** l'on effectue la détermination en utilisant au moins un aptamère et/ou un anticorps dirigé contre la prostaglandine-D-synthase.

7. Procédé selon la revendication 6, **caractérisé en ce que** l'on utilise un anticorps polyclonal et/ou monoclonal.

8. Procédé selon l'une des revendications 6 ou 7, **caractérisé en ce que** l'on utilise un anticorps qui peut être obtenu à l'aide d'au moins un des peptides PGDS1, PGDS2 et/ou PGDS3.

9. Procédé selon l'une des revendications précédentes, **caractérisé en ce que** l'on effectue la détermination au moyen d'un essai immunologique, en particulier d'un essai de type ELISA en sandwich quantitatif ou d'un transfert de type Western.

10. Procédé selon l'une des revendications précédentes, **caractérisé en ce que** la détection s'effectue à l'aide de la spectroscopie de masse, en particulier de MALDI-TOF, ou par électrophorèse sur gel, en particulier électrophorèse bidimensionnelle sur gel.

11. Utilisation d'un aptamère ou d'un anticorps dirigé contre la prostaglandine-D-synthase (PGDS) pour la préparation d'un agent destiné à la détection d'inflammations des glandes mammaires.
